# EUROPEAN PATENT APPLICATION

(11) **EP 4 272 799 A1**
(43) Date of publication of application: **08.11.2023**
(21) Application number: 22748882.2
(22) Date of filing: 20.01.2022
(51) Int. Cl.: A61M 25/00, A61M 39/08

(54) **TUBE FIXING DEVICE AND CATHETER PROTECTION DEVICE**

(30) Priority: 05.02.2021 CN 202110160407
(71) Applicant: MicroPort NeuroTech (Shanghai) Co., Ltd., Shanghai 201318 (CN)
(72) Inventor: LIU, Qinglong, Shanghai 201318 (CN); LIU, Yunyun, Shanghai 201318 (CN); LUO, Xueli, Shanghai 201318 (CN); CUN, Yuxi, Shanghai 201318 (CN)
(74) Representative: Marks & Clerk LLP
(86) International application number: PCT/CN2022/072971
(87) International publication number: WO 2022/166603

(57) **Abstract**

A catheter retainer and a catheter-protecting device including the catheter retainer are disclosed. The catheter retainer defines a channel configured for passage of a pre-shaped section of a catheter therethrough. The channel includes a first channel section (2001) and a second channel section (2002). The first channel section (2001) is configured for passage of a straight section of a tip of the catheter therethrough, and the second channel section (2002) is configured for passage of a bent section of the tip of the catheter therethrough. The catheter retainer, which includes the first channel section (2001) and the second channel section (2002), is able to ensure that an angle of a pre-shaped end of the catheter can be maintained without decreasing for a long time, allowing an operator to directly use and deploy the catheter at a lesion site without having to subjecting it to a steam shaping process. This can reduce the time required for preoperative preparation.

## Description

### TECHNICAL FIELD

The present invention relates to the field of medical devices, and in particular to a catheter retainer and a catheter-protecting device including the catheter retainer.

### BACKGROUND

In vascular interventional therapy, in order to deliver therapeutic devices, therapeutic agents and implants to lesion sites, it is necessary to create vascular accesses using combinations of sheaths, guide catheters and microcatheters of various sizes. When a microcatheter is used with a relatively small guide catheter or intermediate catheter (e.g., a 5F or 6F catheter) with a softer tip to create an access for delivery to an intended site, in order to allow an operator to manipulate the catheter to enable it more smoothly pass through possibly continuous, tortuous blood vessels, before being deployed, the catheter is usually steam-shaped and thereby bent at a certain angle at the tip. In addition, in clinical use, a guide catheter or intermediate catheter with a large lumen may get jammed when passing through the ophthalmic artery at the carotid siphon, affecting surgical operation and efficiency. In order to overcome this, the operator would likewise subject the catheter tip to steam shaping in advance for bending it at a certain angle, which can facilitate passage through the ophthalmic artery and improve surgical efficiency.

In order to provide operators' convenience and increased efficiency in interventional procedures which are just like races against time, some manufacturers of microcatheters, guide catheters or intermediate catheters additionally provide angled versions of their catheter products, i.e., catheters with tips that have been bent at predetermined angles before they are delivered from the factory. Such catheters can be directly inserted into a blood vessel to create a vascular access without requiring an operator's steam shaping operation before the procedure.

After delivery from the factory, throughout the shelf life of such catheters with angled tips, the soft catheter tip will experience creep deformation, leading to the tip angle gradually decreasing over time. Consequently, when provided to operators for use, the angle may become too small to be suitable for direct use.

### SUMMARY OF THE INVENTION

In view of the above-discussed disadvantages of the prior art, it is an object of the present invention to provide a catheter retainer, which overcomes one or more of the disadvantages.

To this end, the present invention lies in:
a catheter retainer defining a channel configured for passage of a pre-shaped section of a catheter therethrough, the channel including a first channel section and a second channel section, the first channel section configured for passage of a straight section of a tip of the catheter therethrough, the second channel section configured for passage of a bent section of the tip of the catheter therethrough,
the second channel section and the first channel section forming an angle therebetween.

Additionally, the second channel section and the first channel section may form a linear or arcuate angle therebetween.

Additionally, the first channel section and the second channel section may form a first angle of deflection therebetween, which is not less than a pre-shaped angle of a pre-shaped end of the catheter.

Additionally, the first channel section and the second channel section may define a J-shaped channel.

Additionally, at least two second channel sections may be included.

Additionally, the second channel sections may be parallel to one another.

Additionally, the catheter retainer may include at least one retention portion and define at least one channel, each of which is at least partially disposed within a respective one of the at least one retention portion.

Additionally, the catheter retainer may further include a tubular portion detachably connected or integrally joined to the retention portion, wherein the first channel section extends through a lumen defined in the tubular portion.

Additionally, the lumen may have a diameter varying or remaining constant across its axial length, and the tubular portion may have an outer diameter varying or remaining constant across its axial length.

Additionally, the channel may be circumferentially closed or open on one side.

Additionally, the channel may have any one of circular, rectangular, elliptical, triangular, semicircular and arcuate cross-sectional shapes.

Additionally, the channel may have a diameter varying or remaining constant across its axial length.

Additionally, one side of the channel may be circumferentially provided with an opening, wherein at least one retention sheet is provided at the opening along an axial direction of the channel in order to prevent the catheter from dislodgement from the channel.

Additionally, a width ratio of the retention sheet to the opening may range from 1/3 to 1.

Additionally, the catheter retainer may include a first retention element and at least one second retention element disposed adjacent the first retention element, the first retention element and the second retention element defining a retention channel therebetween configured for engagement and retention of a pre-shaped end of the catheter therein.

Additionally, a width of the retention channel defined between the first retention element and the second retention element may be 1 to 3 times a diameter of the pre-shaped end of the catheter.

Additionally, the first retention element may include at least two portions which are joined together or not, wherein axes of the portions intersect to form a second angle of deflection, which is not less than a pre-shaped angle of the pre-shaped end of the catheter.

Additionally, the second retention element may be a right solid having any one of circular, elliptical, semicircular, rectangular, arcuate and triangular cross-sections.

Additionally, 2-10 second retention elements may be included.

Additionally, the catheter retainer may be made of any one of rubber, a plastic, a metal and a rubber/plastic mixture, or a combination of several thereof.

The present invention also provides a catheter-protecting device including the catheter retainer as defined above and a catheter-protecting tube.

Additionally, the catheter retainer may be integrally joined or detachably connected to the catheter-protecting tube.

Additionally, the catheter-protecting device may further include a backing plate, wherein the catheter retainer is connected to or separate from the backing plate.

Additionally, the catheter retainer may be contiguous with or spaced apart from a tip of the catheter-protecting tube.

Additionally, the catheter-protecting tube may be a coiled or straight tube.

Compared with the prior art, the present invention has the benefits as follows:
1) In the catheter retainer of the present invention, the first channel section is configured for passage of a straight section of a tip of a catheter therethrough, and the second channel section is configured for passage of a bent section of the tip of the catheter therethrough. With this design, it can be ensured that an angle of a pre-shaped end of the catheter is maintained without decreasing for a long time, allowing an operator to directly use and deploy the catheter at a lesion site without having to subjecting it to a steam shaping process. This can reduce the time required for preoperative preparation.
2) Moreover, at least two second channel sections may be provided, which are compatible with pre-shaped catheters of different lengths. Depending on the length of an intended pre-shaped catheter, adjustment can be made to a suitable position.
3) The catheter retainer can avoid a pre-shaped catheter from unnecessary damage and thereby extend its service life. Creep deformation of the catheter tip can be prevented, and higher surgical efficiency can be achieved.

### BRIEF DESCRIPTION OF THE DRAWINGS

Fig. 1 is a schematic diagram showing the structure of a catheter retainer according to a first embodiment of the present invention.
Fig. 2 shows a side view of the catheter retainer according to the first embodiment of the present invention taken in the direction of arrow A.
Fig. 3 is an isometric view of the catheter retainer according to the first embodiment of the present invention.
Fig. 4 is a schematic illustration of a first channel section in the catheter retainer according to the first embodiment of the present invention, which extends throughout a tubular portion.
Fig. 5 shows a side view of the tubular portion in the catheter retainer according to the first embodiment of the present invention taken in the direction of arrow B.
Fig. 6 is a schematic illustration of the first channel section in the catheter retainer according to the first embodiment of the present invention, which extends through part of the tubular portion.
Fig. 7 is a schematic diagram showing the structure of the catheter retainer according to the first embodiment of the present invention, which includes a J-shaped channel.
Fig. 8 is a schematic diagram showing the structure of a catheter retainer according to a second embodiment of the present invention.
Fig. 9 is a schematic diagram showing the structure of a catheter retainer according to a third embodiment of the present invention.
Fig. 10 schematically illustrates a catheter-protecting tube in a catheter retainer according to a fourth embodiment of the present invention, which is fixedly connected to a backing plate.
Fig. 11 is a schematic diagram showing the structure of the catheter retainer according to the fourth embodiment of the present invention.
Fig. 12 shows a side view of the catheter retainer according to the fourth embodiment of the present invention taken in the direction of arrow C.

In these figures:
1-tubular portion; 100-lumen; 2-retention portion; 200-channel; 2001-first channel section; 2002-second channel section; 201-retention sheet; 300-base; 301-first retention element; 3011-first portion; 3012-second portion; 302-second retention element; 303-retention channel; 304-first side groove; 305-second side groove; 4-catheter-protecting tube; 5-backing plate; 501-backing plate fixing belts; 502-opening; 6-catheter.

### DETAILED DESCRIPTION

Objectives, aspects and advantages of the present invention will become more apparent from the following detailed description of catheter retainers according to specific embodiments thereof set forth below when taken in conjunction with the accompanying drawings. Advantages and features of the invention will become more apparent from the following description. Note that the figures are provided in a very simplified form not necessarily drawn to exact scale and for the only purpose of facilitating easy and clear description of the disclosed embodiments. In order that objects, features and advantages of the present invention may become readily apparent, reference is made to the accompanying drawings. It should be noted that architectural, proportional, dimensional and other details in the figures are presented only for the purpose of facilitating, in conjunction with the disclosure herein, the understanding and reading of those familiar with the art rather than being intended to limit conditions under which the present invention can be implemented. Therefore, they are technically of no substantive significance, and any and all architectural modifications, proportional variations or dimensional changes that do not affect the benefits and objects of the present invention are considered to fall within the scope of the teachings herein.

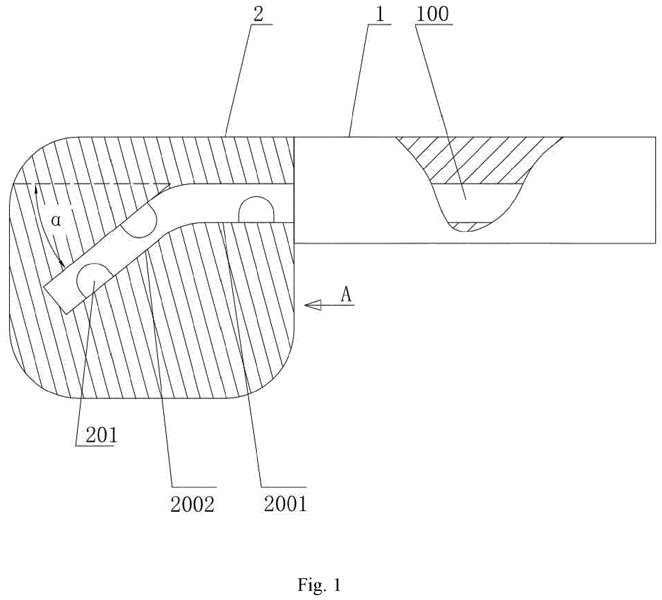

As used herein and in the appended claims, the singular forms "a", "an" and "the" include plural referents unless the context clearly dictates otherwise. As used herein and in the appended claims, the term "or" is employed in the sense including "and/or" unless the context clearly dictates otherwise. Additionally, the terms "proximal" and "distal" are generally used to refer to an end closer to an operator and an end closer to a lesion site in a patient, respectively. For example, the device shown in Fig. 1 has proximal end on the right and a distal end on the left.

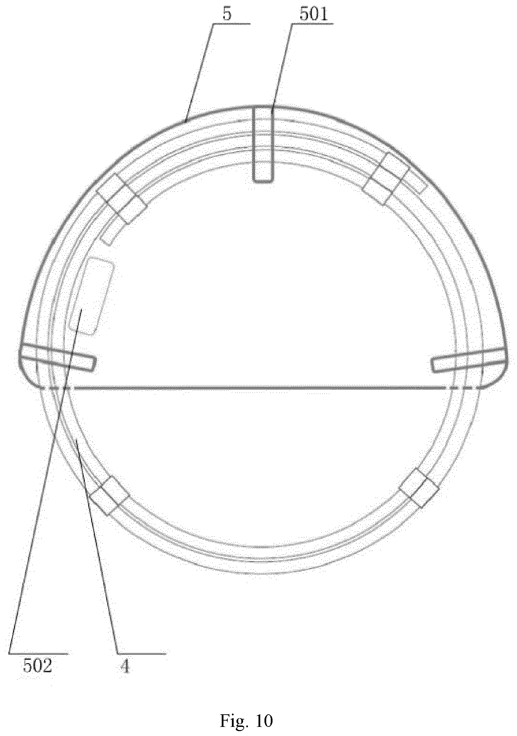

In essence, the present invention provides a catheter retainer mounted essentially at a distal end of a catheter-protecting tube 4, which is usually provided in the form of a coiled spiral tube (see Fig. 10) or an uncoiled straight tube. Before delivery from the factory, in order to avoid possible damage, a catheter is usually received within a lumen of the catheter-protecting tube 4, with a pre-shaped end thereof extending outside of a distal end of the catheter-protecting tube 4 and being connected to the catheter retainer. The catheter retainer may fit on either the inside or outside of the catheter-protecting tube 4 across an axial length thereof, which is adjustable. Alternatively, the catheter retainer may be separate from, abut against or disposed away from the distal end of the catheter-protecting tube 4.

The structure of a catheter retainer according to a first embodiment is detailed below.

Referring to Figs. 1 to 6, in the first embodiment, the catheter retainer is a first structure nested with a pre-shaped end of a catheter. The first structure may fit on either the inside or outside of, and be thereby connected to, a distal end of the catheter-protecting tube 4. The first structure includes a retention portion 2 and a tubular portion 1 joined to the retention portion 2. The tubular portion 1 is configured for passage of the catheter therethrough, optionally, the tubular portion 1 is configured for connection with the distal end of the catheter-protecting tube 4. The retention portion 2 is configured to retain the pre-shaped end of the catheter so as to maintain its pre-shaped angle. The pre-shaped angle refers to an angle of deflection of a tip with respect to a main body of the catheter, which is formed in a pre-shaping process.

In addition, the tubular portion 1 may be detachably coupled to, or integrally formed with, the retention portion 2. In particular, the tubular portion 1 may be integrally formed with the retention portion 2 by injection molding or adhesive bonding. In the catheter retainer of the first embodiment, the tubular portion 1 and the retention portion 2 are integrally joined with each other.

The structure of the tubular portion 1 is detailed below.

Referring to Fig. 1, the tubular portion 1 is any one of cylinder, a truncated cone, a triangular prism or a cuboid and defines a lumen 100. The lumen 100 is preferred to have a circular cross-sectional shape, and the lumen 100 is preferred to have a diameter of 2 mm to 8 mm. Preferably, the tubular portion 1 has an axial length of 10 mm to 100 mm. In some other embodiments, the lumen 100 has any one of a circular, rectangular, elliptical, triangular, semicircular, arcuate, polygonal or other irregularly-shaped cross-section. In this embodiment, the lumen 100 has a constant diameter across the axial length of the tubular portion 1. Moreover, the tubular portion 1 also has a constant outer diameter across its axial length. The diameter of the lumen 100 of the tubular portion 1 matches an outer diameter of the catheter-protecting tube 4. A proximal end of the tubular portion 1 may be disposed over the outside of the distal end of the catheter-protecting tube 4, or spaced apart from the catheter-protecting tube 4. In the catheter retainer of the first embodiment, the tubular portion 1 has an axial length of 20 mm and the lumen 100 is preferred to have a circular cross-sectional shape. Moreover, the tubular portion 1 is a cuboid having a square cross-section with a side length of 7.3 mm. In addition, the circular lumen has a diameter of 5.7 mm. It is to be noted that, as shown in Fig. 1, the four corners of the cuboid tubular portion 1 may be rounded, and accordingly, its cross-section may be a rounded square. In some other embodiments, the tubular portion 1 may be a rounded triangular prism, or an unrounded cuboid or triangular prism.

With continued reference to Fig. 1, the tubular portion 1 is detachably disposed over the outside of the distal end of the catheter-protecting tube 4. Additionally, when the tubular portion 1 is detachably disposed over the outer circumference of the distal end of the catheter-protecting tube 4, the cross-sectional shape of the lumen 100 of the tubular portion 1 matches that of the distal end of the catheter-protecting tube 4. In other embodiments of the present invention where the tubular portion 1 detachably fits on the inside of the distal end of the catheter-protecting tube 4, the cross-sectional shape of the tubular portion 1 may match that of the lumen of the distal end of the catheter-protecting tube 4.

In other embodiments of the present invention, the tubular portion 1 may have an outer diameter gradually decreasing from its proximal end to its distal end across its axial length, or the tubular portion 1 may have an outer diameter gradually decreasing from its distal end to its proximal end across its axial length, and a ratio of the outer diameter values at the proximal and distal ends may range from 0 to 50%.

In other embodiments of the present invention, the lumen 100 of the tubular portion 1 may also have a varying diameter, as long as a minimum value of the diameter of the lumen 100 allow insertion of the catheter therethrough.

The structure of the retention portion 2 is detailed below.

With continued reference to Fig. 1, the retention portion 2 has a first nesting feature configured for passage of the pre-shaped end of the catheter therethrough.

With continued reference to Fig. 1, the retention portion 2 may be shaped as any one of a cuboid, a cylinder, an elliptical cylinder or a triangular prism and may have a height of 2 mm to 10 mm. Each of side lengths of a rectangular cross-section of the cuboid, a diameter of a circular cross-section of the cylinder, long and short axes of an elliptical circular cross-section of the elliptical cylinder and side lengths of a triangular cross-section of the triangular prism may range from 10 mm to 50 mm. In the first embodiment, the retention portion 2 is a cuboid rounded at the corners and has a size of 18 mm * 15 mm * 4 mm (length * width * height).

Of course, in other embodiments of the present invention, the retention portion may also be a tubular structure having inner and outer diameters, each of which may be kept constant or vary across its axial length.

With continued reference to Fig. 1, in the catheter retainer of the first embodiment, the first nesting feature is a channel formed in the retention portion 2, the proximal end of the channel is located at a proximal end of the retention portion 2 and the distal end of the channel is positioned within the retention portion 2 at a predetermined depth of 1 mm to 5 mm. The channel has a width of 1 mm to 5 mm. Specifically, the proximal end of the channel is open in a proximal end face of the retention portion 2 and distal end of the channel extends toward a distal end of the retention portion 2 over a predetermined depth. The depth of the channel 200 may be 3.8 mm, and the width of the channel 200 may also be 3.8 mm.

In other embodiments of the present invention, the distal end of the channel may be positioned within the retention portion 2 and extend through the entire retention portion 2, as long as it can be used to retain the pre-shaped end of the catheter.

In other embodiments of the present invention, inner diameters of the channel may vary in an axial direction of the retention portion 2, and outer diameters of the channel may vary in an axial direction of the retention portion 2, as will be further detailed below in the second embodiment.

With continued reference to Fig. 1, the channel includes a first channel section 2001 and a second channel section 2002. The first channel section 2001 deflects at an angle relative to the second channel section 2002, which is defined by a rounded corner. That is, the rounded corner provides an arc-shaped transition between the two sections. The first channel section is configured for passage of a straight section of the catheter tip therethrough, and the second channel section is configured for passage of a bent section of the catheter tip therethrough. The first channel section 2001 has a length of 3 mm to 30 mm, and a total length of the first channel section 2001 and the second channel section 2002 ranges from 10 mm to 50 mm. An angle formed between the first channel section 2001 and the second channel section 2002 is defined as a first angle of deflection α, which ranges from 5° to 180°. Preferably, the first angle of deflection α is not less than the pre-shaped angle of the catheter tip. In the retention portion 2 of the first embodiment, the length of the first channel section 2001 is 10 mm, and the total length of the first channel section 2001 and the second channel section 2002 is 17 mm. The first angle of deflection α of the first channel section 2001 relative to the second channel section 2002 is 30°. In some other embodiments, the first angle of deflection α of the first channel section 2001 relative to the second channel section 2002 is between 5° and 180°, such as 5°, 15°, 45°, 60°, 90°, 120°, 150° or 180°.

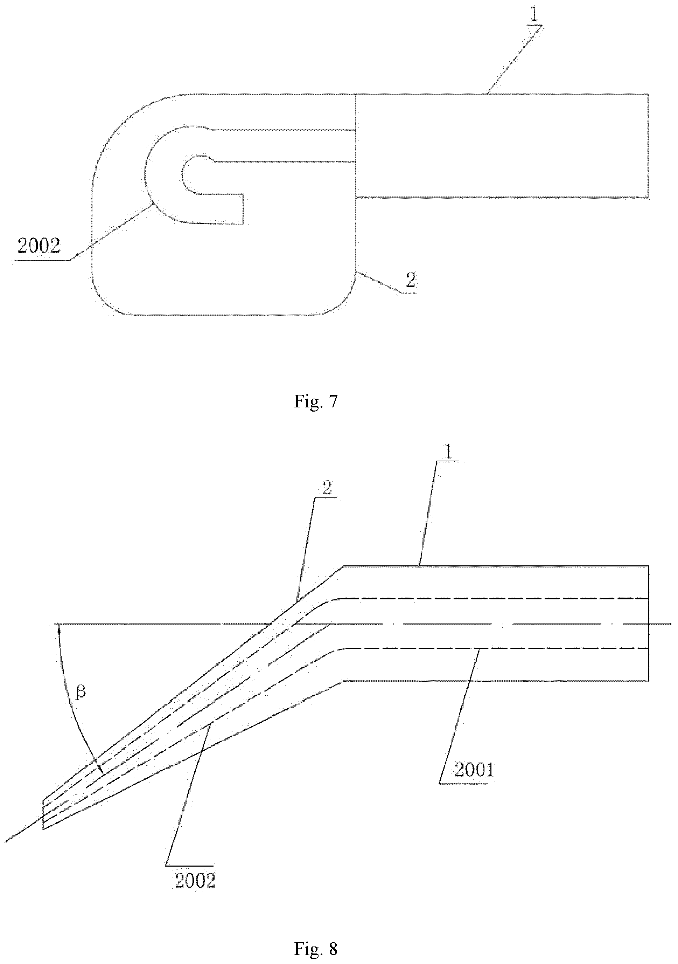

Referring to Fig. 7, in other embodiments of the present invention, the second channel section 2002 may further have a curved segment, which makes the channel consisting of first channel section 2001 and the second channel section 2002 generally appear like the letter "J".

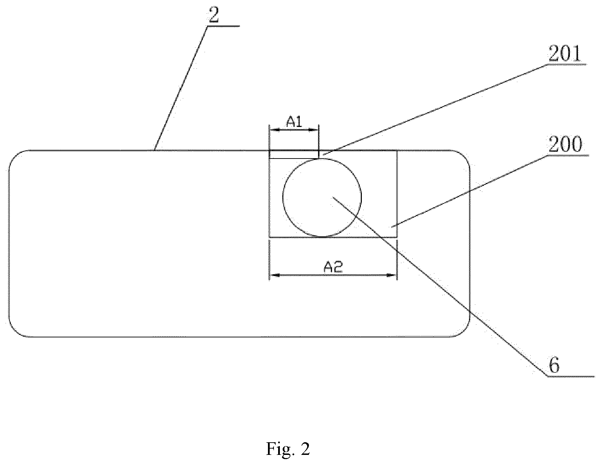

Referring to Figs. 1 and 2, the channel may be closed or open on one side circumferentially. According to embodiments of the present invention, in the catheter retainer, the channel may be circumferentially open on one side, and at least one retention sheet 201 may be provided at the opening, the retention sheet 201 is configured to prevent the catheter 6 from falling out of the channel. The retention sheet 201 may have any one of an elliptical shape, a triangular shape and a semicircular shape. Two to twenty retention sheets 201 may be arranged at interval(s) along an axial direction of the channel. A width A1 of the retention sheet 201 may be 1/3 to 100% of a width A2 of the opening. Specifically, in the first embodiment of the present invention, the retention sheet 201 is preferred to have a semicircular shape, there are 3 retention sheets 201 and the retention sheets 201 are arranged at intervals along the axial direction of the channel, and the width of the retention sheets 201 is 2/3 of the width of the opening.

In the catheter retainer of the first embodiment, the retention sheets 201 are arranged in a single plane. However, in other embodiments of the present invention, the retention sheets 201 may be staggered from one another and arranged in different planes. The retention sheets 201 may be arranged on the same or different sides of the opening of the channel, as long as they can stop the catheter and prevent its dislodgement from the channel.

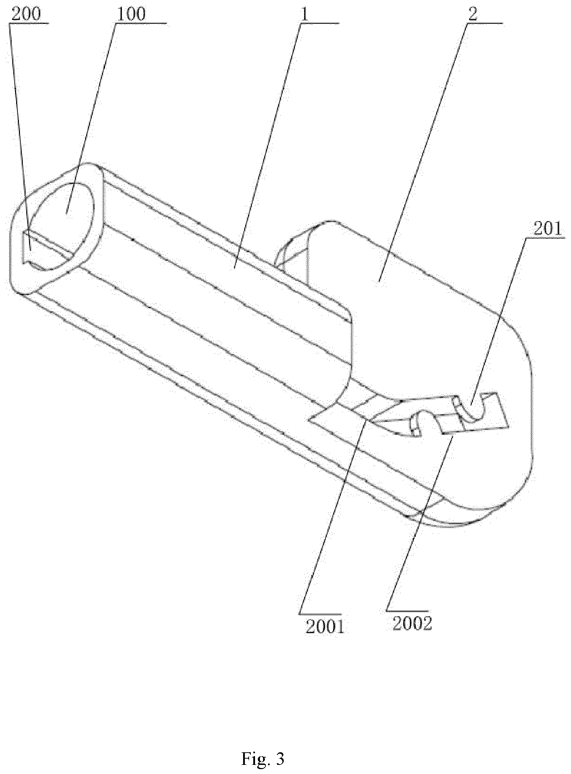

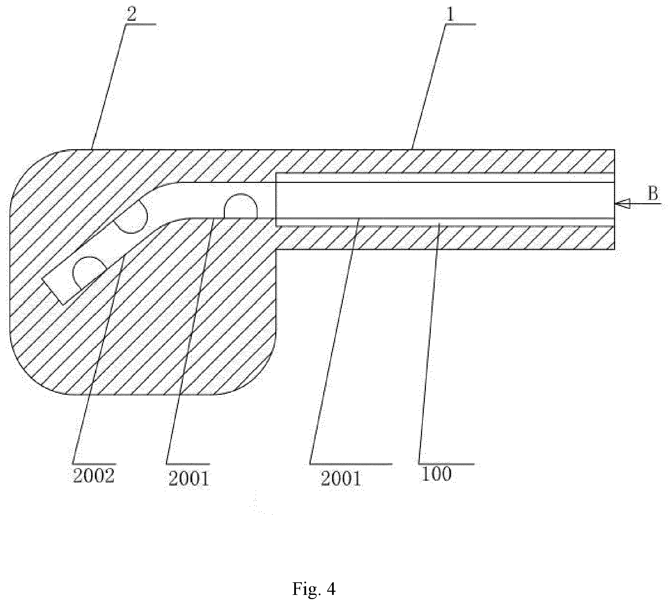

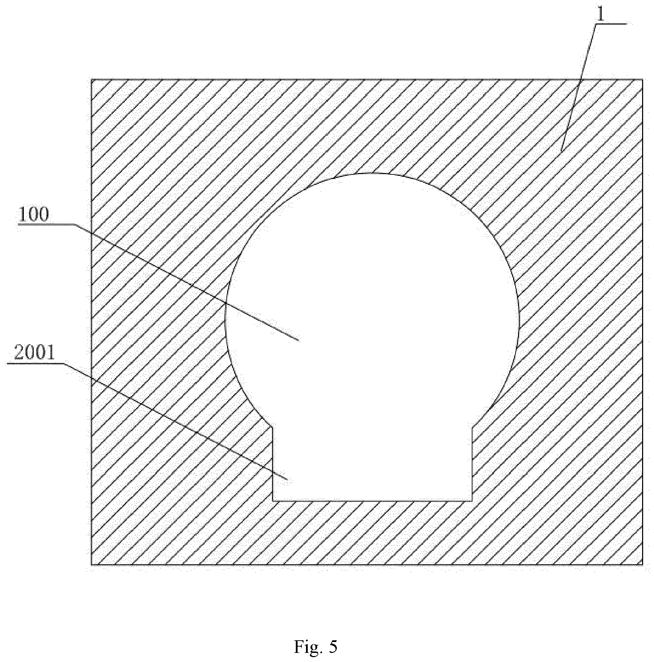

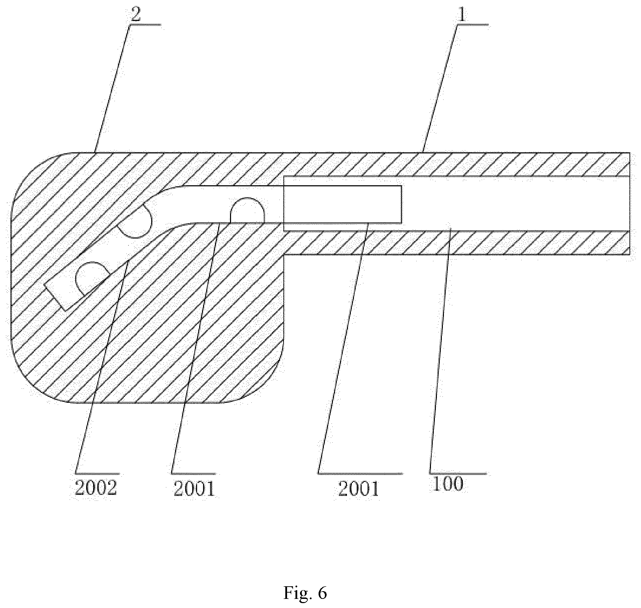

In other embodiments of the present invention, the first channel section 2001 of the channel may extend through the entire tubular portion 1 or part thereof. When the first channel section 2001 of the channel may extend to the entire tubular portion 1, the channel may have a total axial length of 20 mm to 150 mm. Fig. 4 schematically shows the first channel section 2001 of the channel, which extends throughout the tubular portion, and Fig. 5 is a side view of the tubular portion taken the direction of arrow B. Fig. 6 schematically shows the first channel section 2001 of the channel, which extends through part of the tubular portion. Referring to Figs. 3 and 4, in case of the first channel section 2001 of the channel extending throughout the tubular portion 1, the total axial length of the channel is preferred to be 30 mm. Through designing the channel so that the first channel section 2001 extends through the entire tubular portion 1 or part thereof, when an excessive length of the catheter 6 extends out of the catheter-protecting tube 4, an excessive portion of the catheter 6 may extend and be retained within the first channel section 2001 of the channel in the tubular portion 1, enabling the catheter 6 to be retained in the first channel section 2001 over a larger length.

Differing from the catheter retainer of this embodiment that includes both the tubular portion 1 and the retention portion 2, in some other embodiments, the catheter retainer may include only the retention portion 2. In these cases, the catheter retainer may be spaced apart from the distal end of the catheter-protecting tube.

Differing from the retention portion 2 of this embodiment that defines one channel including one first channel section 2001 and one second channel section 2002, in some other embodiments, the retention portion 2 defines two or more channels each including one first channel section 2001 and one second channel section 2002. In some other embodiments, the retention portion 2 may define at least one channel each including one first channel section 2001 and at least two second channel sections 2002. The number of second channel sections 2002 may be in the range of 2 to 20, such as 2, 3, 5, 8, 10, 12, 15, 18, 20, etc. These second channel sections 2002 may be parallel or not. In some other embodiments, the retention portion may include at least one second channel section 2002.

Further, the tubular portion 1 and the retention portion 2 may be made of any one of rubber, a plastic, a metal and a rubber/plastic mixture, or a combination thereof.

The plastic may be any one of polyolefin (PO), polyethylene (PE) and polypropylene (PP).

The rubber/plastic mixture may be any one of a thermoplastic elastomer (TPE), thermoplastic polyurethane (TPU), thermoplastic polyamide (TPA) and polyether block amide copolymer (Pebax).

The catheter retainer according to the first embodiment of the present invention may be used as follows:
Referring to Figs. 1 and 2, the catheter 6 is inserted through the catheter-protecting tube 4 so that the pre-shaped end of the catheter 6 protrudes out of the distal end of the catheter-protecting tube 4 and enters the lumen 100. The pre-shaped end of the catheter 6 is further advanced until it passes through the first channel section 2001 into the second channel section 2002. In this way, the pre-shaped end of the catheter 6 is retained within the second channel section 2002 of the channel. Subsequently, the catheter retainer that retains the pre-shaped end of the catheter is fitted on either the inside or outside of the distal end of the catheter-protecting tube 4 and adjusted to a suitable position in an axial direction of the catheter-protecting tube 4.

The structure of a catheter retainer according to a second embodiment is detailed below.

The catheter retainer of the second embodiment is substantially similar to that of the first embodiment of the present invention. Only different features of the second embodiment are described below, and further description of any common feature that it shares with the first embodiment is deemed unnecessary and omitted herein.

Referring to Fig. 8, in the catheter retainer of the second embodiment, a tubular portion 1 and a retention portion 2 are integrally joined together by injection molding. The retention portion 2 may be any one of a right solid (e.g., a cylinder, a triangular prism or a cuboid), a frustum or a pointed solid (e.g., a cone). A diameter of a circular cross-section, side lengths of a rectangular cross-section, side lengths of a triangular cross-section, or dimensions of otherwise-shaped cross-section of the retention portion 2 may be not greater than a diameter or any side length of the tubular portion 1 and may range from 3 mm to 12 mm.

With continued reference to Fig. 8, similarly, the catheter retainer of the second embodiment includes a first channel section 2001 and a second channel section 2002. The first channel section 2001 extends within the tubular portion 1 and defines a lumen allowing passage of a catheter therethrough. The second channel section 2002 is provided within the retention portion 2 and configured for passage of a bent section of a tip of the catheter therethrough. The second channel section 2002 has an inner diameter of 2 mm to 8 mm, and a ratio of a diameter of the lumen in the tubular portion 1 to an outer diameter of the catheter 6 ranges from 1.5:1 to 1: 1. The retention portion 2 has a length in the range of 10 mm to 50 mm.

With continued reference to Fig. 8, in the catheter retainer of the second embodiment, the tubular portion 1 has a circular cross-section and an outer diameter preferred to be 3.5 mm. The tubular portion 1 defines a constant lumen with a diameter of 2.3 mm and has an axial length of 30 mm. Moreover, the tubular portion 1 has an outer diameter that is also constant across its entire axial length.

With continued reference to Fig. 8, in the catheter retainer of the second embodiment, the retention portion 2 also has a second nesting feature for retaining a pre-shaped end of the catheter. The second nesting feature is provided by the second channel section 2002. Both an outer diameter of the retention portion 2 and a diameter of the second channel section 2002 gradually decrease from a proximal end to a distal end of the retention portion 2. In the retention portion 2, the distal end of the second channel section 2002 extends through the retention portion 2 (or in some other embodiments, the distal end of the second channel section 2002 does not extend through the retention portion 2) and the proximal end of the second channel section 2002 is joined to the first channel section 2001. The outer diameter at the proximal end of the retention portion 2 is preferred to be 3 mm to 12 mm, and the diameter at the proximal end of the second channel section 2002 is preferred to be 2 mm to 10 mm. The outer diameter of the retention portion 2 is preferred to be 1 mm to 12 mm at the distal end, and the diameter of the second channel section 2002 is preferred to be 0.1 mm to 10 mm at the distal end. The retention portion 2 is preferred to have a length of 10 mm to 50 mm. In the second embodiment, the outer diameter of the retention portion 2 is 3.5 mm at the proximal end, and the diameter of the second channel section 2002 is 2.3 mm at the proximal end. Moreover, the outer diameter of the retention portion 2 is 2.0 mm at the distal end, and the diameter of the second channel section 2002 is 0.8 mm at the distal end. Additionally, the length of the retention portion 2 is 20 mm. In the second embodiment, the retention portion 2 deflects relative to the tubular portion 1. Here, the deflection means that, unlike the first embodiment in which axes of the retention portion 2 and the tubular portion 1 extends in the same direction, in this embodiment, an angle of deflection β is formed between the axes of the retention portion 2 and the tubular portion 1. The angle of deflection β may range from 5° to 180°. In the second embodiment, the angle of deflection β is preferred to be 40°. Further, a first angle of deflection is formed between the first channel section 2001 in the tubular portion 1 and the second channel section 2002 in the retention portion. The first angle of deflection may be equal to the angle of deflection β, or not. Preferably, the first angle of deflection is equal to the angle of deflection β. The first angle of deflection is preferred to be 40°. In some other embodiments, the first angle of deflection and/or the angle of deflection β may be 5° to 180°, such as 5°, 15°, 45°, 60°, 90°, 120°, 150° or 180°.

In this embodiment, the tubular portion 1 may proximally fit on either the inside or outside of a distal end of a catheter-protecting tube 4. In some other embodiments, the tubular portion 1 may be spaced apart from the catheter-protecting tube 4. In this embodiment, both the tubular portion 1 and the retention portion 2 are circumferentially closed. In some other embodiments, the tubular portion 1 and/or the retention portion 2 may be circumferentially open across its entire length or part thereof.

With continued reference to Fig. 8, during packaging and use, the pre-shaped end of the catheter 6 is inserted through a lumen 10 of the tubular portion 1 (defined by the first channel section 2001 in this embodiment) and into the second channel section 2002 in the retention portion 2 until it is totally received and retained in the second channel section 2002. After that, the catheter retainer is disposed over the outside of the distal end of the catheter-protecting tube 4, adjusted to a suitable axial position and fixed there.

The structure of a catheter retainer according to a third embodiment is detailed below.

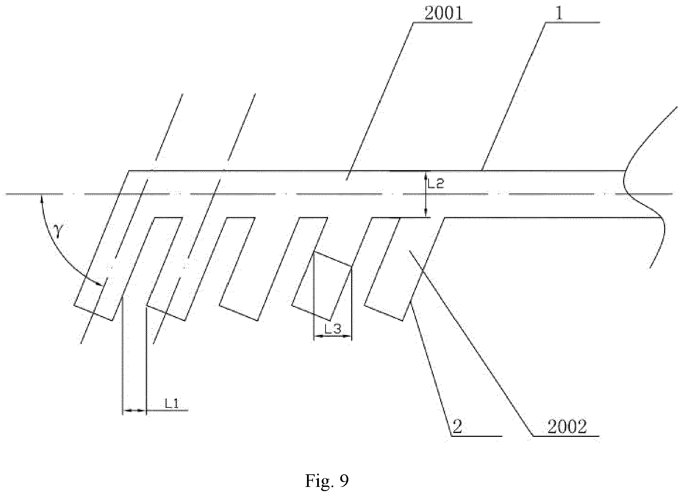

Referring to Fig. 9, in the third embodiment, the catheter retainer is detachably arranged with respect to a tip of the catheter-protecting tube 4 and spaced apart therefrom. The catheter retainer is fixedly connected to a backing plate 5, particularly by means of any one of adhesive bonding, hot-melt welding or a fastener. In this embodiment, the catheter retainer is glued to the backing plate 5. In other embodiments of a similar structure as this embodiment, the catheter retainer may also proximally fit on either the inside or outside of a distal end of the catheter-protecting tube.

With continued reference to Fig. 9, similar to the second embodiment, the catheter retainer of the third embodiment also has a first channel section 2001 and second channel sections 2002. The first channel section 2001 is provided in a tubular portion 1 (a lumen thereof servers as the first channel section 2001, in this embodiment), and the multiple second channel sections 2002 correspond to respective retention portions 2. Adjacent second channel sections 2002 are parallel to each other. The retention portions 2 are integrally joined to the tubular portion 1. The first channel section 2001 in the tubular portion 1 is configured for a straight section of a tip of a catheter therethrough, while the second channel sections 2002 in the retention portions 2 are configured for a bent section of the tip of the catheter therethrough.

Differences of the third embodiment from the second embodiment are detailed below.

The multiple retention portions 2 joined to the tubular portion 1 are parallel to and spaced apart from one another. Each retention portion 2 deflects with respect to the tubular portion 1 so that an angle of deflection γ is formed between center axes of the retention portion 2 and the tubular portion 1. Moreover, a first angle of deflection is formed between the first channel section 2001 in the tubular portion 1 and the second channel section 2002 in the retention portion 2. The first angle of deflection may be equal to the angle of deflection γ, or not. Preferably, the first angle of deflection is equal to the angle of deflection γ (i.e., the first channel section 2001 is parallel to the center axis of the tubular portion 1, while the second channel section 2002 is parallel to the center axis of the retention portion 2).

With continued reference to Fig. 9, additionally, the angle of deflection γ and/or the first angle of deflection may range from 5° to 180°. In the third embodiment, the angle of deflection γ and the first angle of deflection are preferred to be 60°. The number of retention portions 2 may range from 2 to 20. In the third embodiment, preferably, 5 retention portions 2 are provided. In some other embodiments, the number of retention portions 2 or second channel sections 2002 may be 2, 3, 8, 10, 12, 15, 18, 20, etc.

The tubular portion 1 may have a length of 10 mm to 100 mm, and each retention portions 2 may have a length of 10 mm to 50 mm. Adjacent retention portions 2 may be spaced apart at an interval L1 of 1 mm to 5 mm. The tubular portion 1 may have a width L2, which may be equal to a width L3 of each retention portion 2, or not.

Further, in the catheter retainer of the third embodiment, both the tubular portion 1 and the retention portions 2 may be made of a thermoplastic polyamide (TPA). Of course, in other embodiments of the present invention, the tubular portion 1 and the retention portions 2 may be made of different materials.

In some other embodiments, the first channel section 2001 and at least one second channel section 2002 may be provided by several projections.

During packaging and use, the catheter 6 is so mounted in the catheter-protecting tube 4 as to extend out of the distal end of the catheter-protecting tube 4 into a lumen (not shown) of the tubular portion 1. The second channel section 2002 in the retention portion 2 at a compatible position may be selected depending on a length of the catheter 6, that is, a pre-shaped end of the catheter may be selectively inserted in the second channel section 2002 in any of the retention portions 2. After being inserted in the second channel section 2002, the pre-shaped end of the catheter may be retained. Afterwards, the catheter retainer may be overall glued to the backing plate 5 at an opening 502 thereof.

The structure of a catheter retainer according to a fourth embodiment is detailed below.

In the fourth embodiment, the catheter retainer is similarly disposed at a distal end of a catheter-protecting tube 4 and also has a first channel section 2001 and a second channel section 2002.

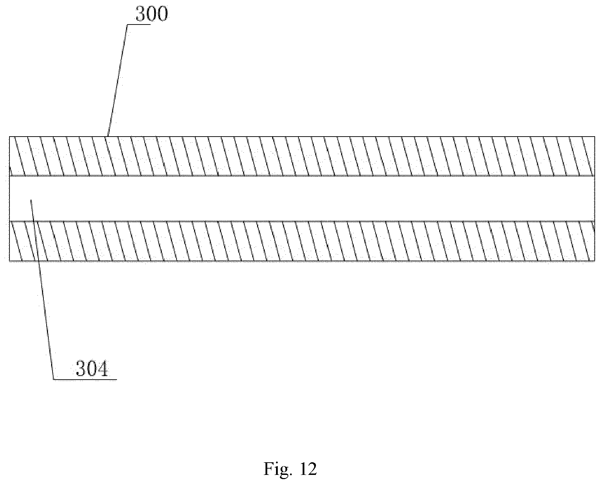

Referring to Figs. 10 to 12, the catheter retainer includes a first retention element 301 and a second retention element 302, which is joined to and disposed adjacent the first retention element 301. While one second retention element 302 is provided in the fourth embodiment, the present invention is not so limited, and in alternative embodiments, one or more second retention elements 302 may be provided, e.g., 3, 5, 8, etc. The first retention element 301 and the second retention element 302 define a retention channel 303 therebetween, the retention channel 303 is configured for firmly retaining a pre-shaped end of a catheter therein.

Of course, in other embodiments of the present invention, the first retention element 301 and the second retention element 302 may be separate from each other, as long as it is ensured that a desired retention channel can be defined between the first retention element 301 and the second retention element 302. Moreover, a gap between the first retention element 301 and the second retention element 302 should not be greater than an outer diameter of the catheter in order to prevent the catheter from passing through the gap.

With continued reference to Figs. 10 to 12, the structure of the first retention element 301 is first detailed below.

The first retention element includes a first portion 3011 and a second portion 3012. An axis of the first portion 3011 intersects an axis of the second portion 3012, forming a second angle of deflection θ between the first portion 3011 and the second portion 3012, which ranges from 5° to 180°. In the fourth embodiment, the first retention element 301 consists of the first portion 3011 and the second portion 3012 that are joined to each other. The first channel section is defined between an inner side of the second portion 3012 and the outside of the second retention element 302, and the second channel section is defined between an inner side of the first portion 3011 and the outside of the second retention element 302. The second angle of deflection θ formed at the joint of the first portion 3011 and the second portion 3012 is preferred to be 90°. The first retention element 301 has a total length of 10 mm to 40 mm, a height of 2 mm to 20 mm and a width of 1 mm to 10 mm. In the fourth embodiment, the length of the first retention element 301 is preferred to be 40 mm, the height is preferred to be 5 mm, and the width is preferred to be 2 mm. A length ratio of the first portion 3011 to the second portion 3012 may range from 1/10 to 2. Preferably, in the fourth embodiment, the lengths of the first portion 3011 and the second portion 3012 are equal.

Of course, in other embodiments of the present invention, with the axes of the first portion 3011 and the second portion 3012 intersecting each other, an arc-shaped transition with a radian of π/18(rad)- π(rad) may be provided at the joint of the first portion 3011 and the second portion 3012.

In alternative embodiments of the present invention, the first portion 3011 and the second portion 3012 may be detachably joined together, as long as the axes of the first portion 3011 and the second portion 3012 can intersect to form the second angle of deflection.

With continued reference to Figs. 10 to 12, the structure of the second retention element 302 is detailed below.

The second retention element 302 may be any right solid with a circular, rectangular, semicircular, elliptical, semielliptic, arcuate or triangular cross-section. A diameter of the circular, semicircular, elliptical or semielliptic cross-section and side lengths of the triangular cross-section are preferred to range from 1 mm to 10 mm. In this embodiment, the second retention element 302 is a right solid with a circular cross-section having a diameter of 2 mm. Moreover, the right solid has a height of 10 mm. The height of the right solid may range from 2 mm to 20 mm. In the fourth embodiment, the circular main body has a height of 10 mm.

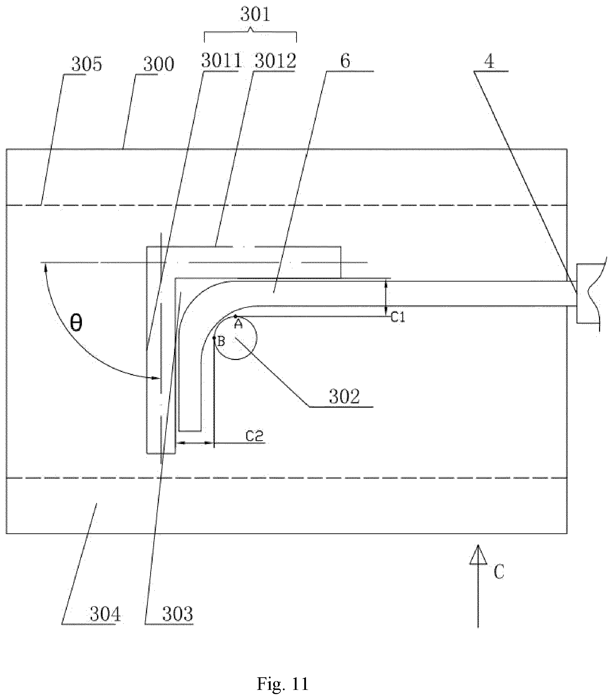

With continued reference to Figs. 10 to 12, the retention channel 303 defined between the first retention element 301 and the second retention element 302 has a width that is 1 to 3 times a diameter of the catheter 6. Specifically, referring to Fig. 11, the width of the retention channel 303 refers to a first distance C1 measured from a first tangent point A of the second retention element 302 to an inner side of the second portion 3012, or a second distance C2 measured from a second tangent point B of the second retention element 302 to an inner side of the first portion 3011. Both the first distance C1 and the second distance C2 may be equal to a minimum distance between the first retention element 301 and the second retention element 302. Alternatively, the first distance C1 and the second distance C2 may be unequal. In the case of the first distance C1 and the second distance C2 being unequal, the first distance C1 may be alternatively defined as a distance measured along the direction of a non-longitudinal axis to the inner side of the first portion 3011, and the second distance C2 may be similarly defined as a distance measured along the direction of a non-transverse axis to the inner side of the second portion 3012, as long as the retention of the pre-shaped end of the catheter can be achieved. In this embodiment, the minimum distance between the first retention element 301 and the second retention element 302, i.e., the first distance C1 or the second distance C2, is 1.5 mm.

Referring to Figs. 10 to 12, in the fourth embodiment, the catheter retainer is not directly connected to the catheter-protecting tube 4. Rather, the catheter-protecting tube 4 is detachably connected to a backing plate 5, and the catheter retainer is movably coupled to the backing plate 5 via an adjustment mechanism. In this way, the catheter retainer can be adjusted in position relative to the backing plate 5 by manipulating the adjustment mechanism.

With continued reference to Figs. 10 to 12, the backing plate 5 may be made of any of a metal, a polymeric material or another high-hardness material (e.g., hard cardboard). Several backing plate fixing belts 501 may be scattered along an axial direction of the backing plate 5 at a peripheral edge thereof. The backing plate fixing belts 501 can be opened and closed relative to the backing plate 5. When closed, the backing plate fixing belts 501 can cover portions of the catheter-protecting tube 4 and be connected to the backing plate 5, thereby securing the catheter-protecting tube 4. On the contrary, when the backing plate fixing belts 501 are opened, the catheter-protecting tube 4 will be released.

With continued reference to Figs. 10 to 12, the adjustment mechanism includes an opening 502 and a base 300 which can be movably connected to the opening 502. The opening 502 is formed in a surface of the backing plate 5, and the base 300 is configured to fix the first retention element 301 and the second retention element 302. The base 300 may be any one of a cuboid or a cylinder. In case of the base 300 being implemented as a cuboid, it may have a size of (20-60 mm) * (20 -60 mm) * (5-40 mm) (length * width * height). In case of the base 300 being implemented as a cylinder, it may have a cross-sectional diameter of 10 mm to 60 mm. In the catheter retainer of the fourth embodiment, the base 300 is preferred to a cuboid, and a first side groove 304 and a second side groove 305 are formed in opposite front and back side faces of the cuboid, respectively. The first side groove 304 and the second side groove 305 may have a depth of 1 mm to 10 mm and a width of 1 mm to 10 mm. In the fourth embodiment, the depth of the first side groove 304 is 5 mm, and its width is 2 mm. Edges of the opening 502 may be received respectively within the first side groove 304 and the second side groove 305 so that the base 300 is movable relative to the opening 502 with the aid of the first side groove 304 and the second side groove 305.

In other embodiments of the present invention, the base 300 may not be connected to the backing plate via the opening 502. Instead, for example, the base 300 may be directly bonded to or engaged with the backing plate, and the opening 502 in the backing plate may be omitted.

Further, in the catheter retainer of the fourth embodiment, the first retention element 301 and the second retention element 302 may be made of the same or different materials, each of which is any one of rubber, a plastic, a metal and a rubber/plastic mixture, or a combination thereof.

The plastic may be any one of polyolefin (PO), polyethylene (PE) and polypropylene (PP).

The rubber/plastic mixture may be any one of a thermoplastic elastomer (TPE), thermoplastic polyurethane (TPU), thermoplastic polyamide (TPA) and polyether block amide copolymer (Pebax).

Furthermore, in the catheter retainer of the fourth embodiment, both of the first retention element 301 and the second retention element 302 are made of polyether block amide copolymer (Pebax).

The catheter retainer according to the fourth embodiment of the present invention may be used as follows:
Referring to Figs. 10 to 12, during packaging and use, the pre-shaped end of the catheter 6 is inserted through the catheter-protecting tube 4 so as to protrude out thereof from its distal end. Subsequently, the pre-shaped end of the catheter is further advanced into and retained in the retention channel 303 between the first retention element 301 and the second retention element 302. The catheter retainer that retains the pre-shaped end of the catheter thereon is fitted into the opening 502 in the backing plate 5 by means of the base 300. Edges of the opening 502 are received respectively in the first side groove 304 and the second side groove 305 on the base 300. The base 300 is then moved relative to the opening 502. In this way, the pre-shaped end of the catheter can be retained, and the catheter retainer can be adjusted to a suitable position in an axial direction of the catheter-protecting tube 4.

In a fifth embodiment of the present invention, there is provided a catheter retainer including the catheter retainer and the catheter-protecting tube according to any of the first to fourth embodiments. The catheter retainer is detachably coupled to the catheter-protecting tube. In some other embodiments, the catheter retainer and the catheter-protecting tube may be integrally joined together. In some other embodiments, the catheter retainer may be separated from the catheter-protecting tube. In some other embodiments, the catheter retainer may be separate, and disposed at a distance, from the catheter-protecting tube.

In this embodiment, the catheter-protecting device further includes a backing plate, and the catheter retainer is arranged to be separate from the backing plate. In some other embodiments, the catheter retainer may be connected to the backing plate by engagement, bonding, welding or otherwise.

In this embodiment, the catheter-protecting tube is a coiled tube. In some other embodiments, the catheter-protecting tube may be a straight tube. The catheter-protecting tube is configured to store an unused catheter therein to protect it against any possible damage.

The various technical features of the foregoing embodiments may be combined in any way. Although not all such combinations have been described above for the sake of brevity, any of them is considered to fall within the scope of this specification as long as there is no contradiction between the technical features.

Presented above are merely several embodiments of the present application. Although these embodiments are described with some particularity and in some detail, it should not be construed that they limit the scope of the present application in any sense. It is to be noted that various variations and modifications can be made by those of ordinary skill in the art without departing from the concept of the present application. Accordingly, it is intended that all such variations and modifications are embraced within the scope of this application as defined in the appended claims.

## Claims

1. A catheter retainer, defining a channel configured for passage of a pre-shaped section of a catheter therethrough, the channel comprising a first channel section and a second channel section, the first channel section configured for passage of a straight section of a tip of the catheter therethrough, the second channel section configured for passage of a bent section of the tip of the catheter therethrough,
the second channel section and the first channel section forming an angle therebetween.

2. The catheter retainer of claim 1, wherein the second channel section and the first channel section form a linear or arcuate angle therebetween.

3. The catheter retainer of claim 1, wherein the first channel section and the second channel section form a first angle of deflection therebetween, the first angle of deflection is not less than a pre-shaped angle of a pre-shaped end of the catheter.

4. The catheter retainer of claim 1, wherein the first channel section and the second channel section define a J-shaped channel.

5. The catheter retainer of claim 1, wherein at least two second channel sections are comprised.

6. The catheter retainer of claim 5, wherein the second channel sections are parallel to one another.

7. The catheter retainer of claim 1, comprising at least one retention portion and defining at least one channel, each of which is at least partially disposed within a respective one of the at least one retention portion.

8. The catheter retainer of claim 7, further comprising a tubular portion detachably connected or integrally joined to the retention portion, wherein the first channel section extends through a lumen defined in the tubular portion.

9. The catheter retainer of claim 8, wherein the lumen has a diameter varying or remaining constant across an axial direction and the tubular portion has an outer diameter varying or remaining constant across the axial direction.

10. The catheter retainer of claim 1, wherein the channel is circumferentially closed or open on one side.

11. The catheter retainer of claim 10, wherein the channel has any one of circular, rectangular, elliptical, triangular, semicircular and arcuate cross-sectional shapes.

12. The catheter retainer of claim 10, wherein the channel has a diameter varying or remaining constant across an axial direction.

13. The catheter retainer of claim 10, wherein one side of the channel is circumferentially provided with an opening, wherein at least one retention sheet is provided at the opening, the at least one retention sheet extending along an axial direction of the channel in order to prevent the catheter from dislodgement from the channel.

14. The catheter retainer of claim 13, wherein a width ratio of the retention sheet to the opening ranges from 1/3 to 1.

15. The catheter retainer of claim 1, comprising a first retention element and at least one second retention element disposed adjacent the first retention element, the first retention element and the second retention element defining a retention channel therebetween configured for engagement and retention of a pre-shaped end of the catheter therein.

16. The catheter retainer of claim 15, wherein a width of the retention channel defined between the first retention element and the second retention element is 1 to 3 times a diameter of the pre-shaped end of the catheter.

17. The catheter retainer of claim 15, wherein the first retention element comprises at least two portions which are joined together or not, wherein axes of the portions intersect to form a second angle of deflection, the second angle of deflection is not less than the pre-shaped angle of the pre-shaped end of the catheter.

18. The catheter retainer of claim 15, wherein the second retention element is a right solid having any one of circular, elliptical, semicircular, rectangular, arcuate and triangular cross-sections.

19. The catheter retainer of claim 15, wherein 2-10 second retention elements are comprised.

20. The catheter retainer of claim 1, wherein the catheter retainer is made of any one of rubber, a plastic, a metal and a rubber/plastic mixture, or a combination of several thereof.

21. A catheter-protecting device, comprising the catheter retainer of any one of claims 1 to 20 and a catheter-protecting tube.

22. The catheter-protecting device of claim 21, wherein the catheter retainer is integrally joined or detachably connected to the catheter-protecting tube.

23. The catheter-protecting device of claim 21, further comprising a backing plate, wherein the catheter retainer is connected to or separate from the backing plate.

24. The catheter-protecting device of claim 21, wherein the catheter retainer is contiguous with or spaced apart from a tip of the catheter-protecting tube.

25. The catheter-protecting device of claim 21, wherein the catheter-protecting tube is a coiled or straight tube.
